# EUROPEAN PATENT SPECIFICATION

(11) **EP 0 581 589 B1**
(45) Date of publication and mention of the grant of the patent: **26.03.2003**
(21) Application number: 93305974.3
(22) Date of filing: 28.07.1993
(51) Int. Cl.: C07D 405/14, B41M 5/145

(54) **Phthalide compounds, and recording materials using the compounds**
Phthalide Verbindungen und damit hergestelltes Aufzeichnungsmaterial
Composés phtalidiques et support d'enregistrement les contenant

(30) Priority: 31.07.1992 JP 22472792
(43) Date of publication of application: 02.02.1994
(73) Proprietor: YAMAMOTO CHEMICALS, INC., Yao-shi Osaka-fu (JP)
(72) Inventor: Sawano, Bunji, Minamikawachi-gun, Osaka, 587 (JP); Nakao, Satoshi, Osaka-shi, Osaka, 543 (JP); Horiuchi, Ken-ichiro, Fujiidera-shi, Osaka, 583 (JP); Iwasaki, Yasuhisa, Ikoma-gun, Nara, 636 (JP)
(74) Representative: Perry, Robert Edward

(56) References cited:
- US-A- 4 022 771
- US-A- 4 970 308
- US-A- 5 200 519

## Description

The present invention relates to phthalide compounds which are of value as electron-donating leuco dyes, processes for the production thereof, and recording materials utilizing said phthalide compounds.

Recording materials each utilizing the color-forming reaction between an electron-donating leuco dye (hereinafter referred to briefly as leuco dye) and an electron-accepting color developer (hereinafter referred to briefly as color developer) are well known and particularly pressure-sensitive and thermal recording materials are in widespread use.

Recently, an optical character reader (hereinafter referred to briefly as OCR) employing a light-emitting diode, a semiconductor laser or the like as the light source has been specified with increasing frequency for reading the images recorded on such pressure-sensitive and thermal recording materials. In order that the recorded image may be read with an OCR, it is essential that the image has an absorption in the wavelength region corresponding to the emission wavelength of the light source used in the OCR. While OCR light sources are available in a variety of wavelength ratings, light sources in the range of 650-750 nm are particularly popular,
and this means that the recorded image must absorb light in this wavelength region. As the leuco dyes for use in pressure-sensitive and thermal recording materials, fluorans providing for a black color, such as the compound A shown below, are most frequently used today. However, since the colored form of such a fluoran does not absorb substantially in the wavelength region over 650 nm, the image recorded on a recording material containing the fluoran compound cannot be easily read with an OCR equipped with a light source having an emission wavelength within the range of 650-750 nm.

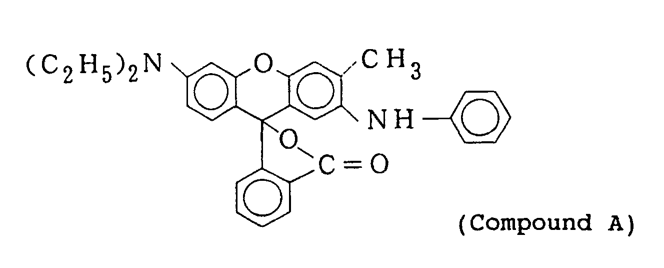

As leuco dyes of which colored forms have an absorption in the wavelength range of 650-750 nm, the phthalide compound B, shown below, has been disclosed in Japanese Tokkyo Koho S-63-5113 and Kokai Tokkyo Koho S-63-165379, for instance. However, compound B and phthalide compounds analogous thereto are colored in yellow-green themselves and, therefore, have the drawback that recording materials utilizing them appear yellow-green in color.

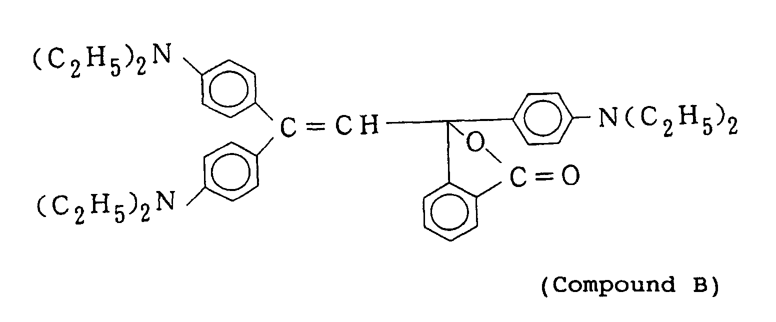

Furthermore, a broad range of similar compounds inclusive of said compound B have been disclosed in Japanese Patent Tokkyo Koho S-58-27825 and the corresponding US Patent 4,022,771.

Novel compounds according to the present invention are the crystal forms defined in claims 1 to 4. Processes for producing these compounds are given in claims 5 and 6. These compounds can be employed as a leuco dye, in recording materials.

The compounds of the invention are each the higher melting crystal modification ("β-crystal modification") of phthalides that exist in two dissimilar crystal structures which have the identical chemical formula. The crystal modification having the lower melting point is described herein as the "α-crystal modification".

When the α-modification of such a phthalide compound is compared with the β-modification which can be represented by the same chemical formula, their color reaction products are alike in absorption characteristics but when they are used in recording materials, particularly thermal recording materials, marked differences are found between them in the degree of whiteness and aging resistance of the background. The degree of whiteness and aging resistance of the background of a recording material utilizing the β-modification are remarkably superior to those of a comparable recording material utilizing the α-modification. The recording material utilizing the α-modification not only has a tendency to be have a tinge of the reaction product color but tends to provide the background with a yellow cast.

A process for producing the compound of the invention comprises a first step of reacting an ethylene compound of general formula (II) with a benzoic acid derivative of general formula (III), preferably in the presence of a dehydrative condensing agent. 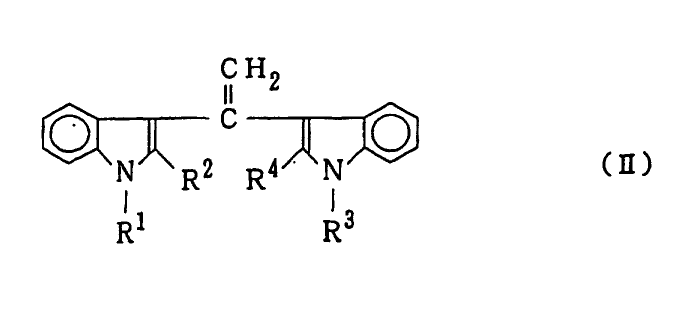 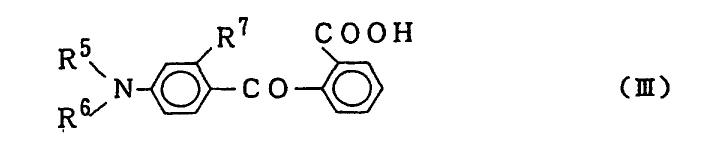 (wherein R¹ and R³ are each ethyl, and R² and R⁴ are each methyl or phenyl (depending on the product), R⁵ and R⁶ are each methyl, ethyl or n-propyl (depending on the product), and R⁷ is H).

The dehydrative condensing agent which can be used includes acetic anhydride, mixture of glacial acetic acid and concentrated sulfuric acid, polyphosphoric acid, phosphorus pentoxide, boron trifluoride, zinc chloride, etc., although acetic anhydride is particularly advantageous.

When acetic anhydride is used as the dehydrative condensing agent, its preferred proportion is not less than 1 mole per mole of the ethylene compound. It is obvious that there is no upper limit to the amount of acetic anhydride that can be used but from economic points of view the use of 1 to 5 mole equivalents is beneficial. Moreover, when an inert solvent such as toluene is used in combination therewith, the use of 1 to 2 mole equivalents is sufficient. The reaction temperature is generally 30 to 120°C and preferably 40 to 80°C. While the reaction time is not critical, the reaction usually goes to completion in several hours.

In conducting the reaction, the concomitant use of an inert solvent such as toluene or dichloroethane is instrumental for insuring a smooth progress of reaction. When the dehydrative condensing agent is acetic anhydride, tolune can be used as the preferred concomitant solvent.

After completion of the reaction, the reaction mixture is subjected to alkali treatment, for example with an aqueous solution of sodium hydroxide, and then extracted with an organic solvent such as toluene for purification. The alkali treatment mentioned above may be conducted in the presence of an organic solvent.

The organic extract is washed with warm water and after phase separation, the organic layer is concentrated and stirred preferably in the presence of methanol on reflux and, then, cooled. In this manner, α-form crystals of phthalide compound are obtained. As an alternative, the organic extract is subjected to a conventional purification procedure, e.g. column chromatography, then concentrated and, where necessary, subjected to the above methanol treatment to obtain α-form crystals or crystals showing no polymorphism.

When the α-modification is heat-treated in the presence of n-hexane or isopropyl alcohol, there is obtained the β-modification.

The heat treatment mentioned above comprises dispersing or dissolving the α-modification in a certain amount of n-hexane or isopropyl alcohol, allowing it to stand for a predetermined time period and, then, cooling the dispersion or solution. The treating temperature may range from 40°C to reflux temperature, preferably from 50°C to reflux temperature. The treating time is 10 minutes to 24 hours, preferably 20 minutes to 10 hours. This treatment is preferably carried out under stirring.

The necessary amount of n-hexane or isopropyl alcohol varies with the specific α-modification of phthalide compound but it is generally 1 to 30 parts by volume, preferably 1 to 20 parts by volume, per part by weight of the α-modification when n-hexane is used and 1 to 20 parts by volume, preferably 1 to 15 parts by volume, per part by weight of the α-modification in the case of isopropyl alcohol.

The β-modification can also be obtained by concentrating the alkali-treated organic extract, adding isopropyl alcohol to the residue and subjecting the mixture to heat treatment.

Where R¹ and R³, and R² and R⁴, respectively, are alike, a two-step one-pot process, that is a serial continuous process consisting of a first step of preparing an ethylene compound and a second step of synthesizing the object phthalide compound from the ethylene compound and a benzoic acid derivative, is commercially advantageous in that the step of isolating the ethylene compound can be omitted. Thus, the phthalide compound can be easily produced by reacting an indole compound of general formula (IV) with acetyl chloride in acetic anhydride and, then, adding a benzoic acid derivative of general formula (III) to the resulting reaction product for further reaction.

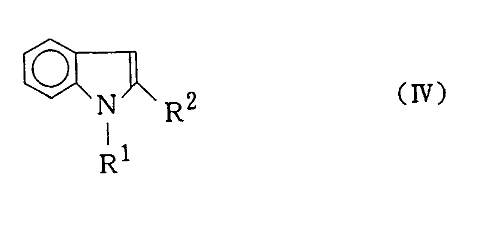

The term 'one-pot process' as used herein means any serial continuous process in which the ethylene compound (II) prepared in the first step is not isolated but is directly reacted with a benzoic acid derivative of general formula (III).

Referring to the first step of such a 2-step, one-pot process, the use of acetic anhydride is not absolutely essential and, therefore, its amount is not critical. However, from procedural points of view, acetic anhydride is preferably used in a proportion of not less than 100mL per mole of the indole compound and from economic points of view, it is preferably used in a proportion of 100 to 200 mL. The use of acetic anhydride offers a further advantage, for the necessary amount of acetyl chloride can then be reduced. Moreover, when acetic anhydride is used in the above-mentioned proportion, it is not particularly necessary to add acetic anhydride in the second step.

The proper amount of acetyl chloride is dependent on the reaction conditions to be used, i.e. the amount of acetic anhydride and the reaction temperature. When acetic anhydride is used in the above proportion and the reaction is conducted at a temperature not below 50°C, the preferred amount of acetyl chloride is not less than 0.2 mole per mole of the indole compound of general formula (IV). There is no upper limit to the amount of acetyl chloride but it suffices that its concentration is not less than 0.2 mole equivalent. However, from economic points of view, acetyl chloride is preferably used in a proportion of 0.2 to 0.5 mole quivalent. If the proportion of acetyl chloride is less than 0.2 mole equivalent, the reaction time will be prolonged or the reaction may not go to completion. The reaction temperature is preferably not lower than 40°C and more advantageously in the range of 50 to 80°C, for the acetyl chloride requirements will generally exceed 1 mole equivalent when the reaction temperature is not higher than 30°C. The reaction time varies with the reactivity of the indole compound and the amount of acetyl chloride but is generally a few to tens of hours.

Since any residue of the acetyl chloride used in Step 1 may interfere with the reaction in Step 2, it is recommendable to inactivate the residual acetyl chloride. The preferred inactivating agent is sodium acetate, calcium acetate or calcium hydroxide and the most preferred is calcium acetate. These inactivating agents may be used in combination. The amount of the inactivating agent is preferably about 1 mole per mole of acetyl chloride used.

Furthermore, the use of a solvent in Step 2 is advantageous in that it assists in effective stirring. As such solvent, toluene can be used with advantage. While there is no particular limitation on the amount of the solvent, it is preferably 0.5 to 2 parts per part of the acetic anhydride used in Step 1.

Of the compounds obtainable by such a two-step, one-pot process in accordance with the present invention, those showing crystal modifications or polymorphism can be subjected to heat-treatment in the presence of n-hexane or isopropyl alcohol as described hereinbefore to provide β-modifications.

The compound of the present invention by itself is substantially colorless but when reacted with a color developer yields a blue-green color and this color reaction product has a maximum absorption in the neighborhood of 680-750 nm. Therefore, the compound of the invention is a leuco dye useful for recording materials suited for image reading with an OCR employing a light source with an emission wavelength of 570-780 nm, particularly 650-750 nm. In particular, the β-crystal form of 3-[2,2-bis(1-ethyl-2-methylindol-3-yl)ethenyl]-3-(4-diethylaminophenyl)phthalide gives a color reaction product showing not less than 80% of absorbance, based on the maximum absorption, over substantially the entire range of 650-750 nm and is, therefore, a leuco dye of value which gives recording materials compatible with OCRs having various emission wavelengths despite its being a single substance.

The absorption of colored form of the compound of the invention, in combination with zinc-modified p-chlorophenyl-formaldehyde resin as a colour developer, was measured. The maximum absorption wavelength, for the four compounds of claims 1 to 4, was respectively 710, 739, 741 and 740 nm. The wavelength range giving not less than 80% of absorbance relative to the absorption maximum for the compounds of claims 1 to 4 was respectively 620-660, 700-770, 700-780 and 700-780 nm.

In recording materials using the colour-forming reaction between a leuco dye and a colour developer, black image reproduction could be accomplished by using one or more species of the compound of the invention as the leuco dye and one or more species of the fluoran compound of general formula (VI) as the color developer. 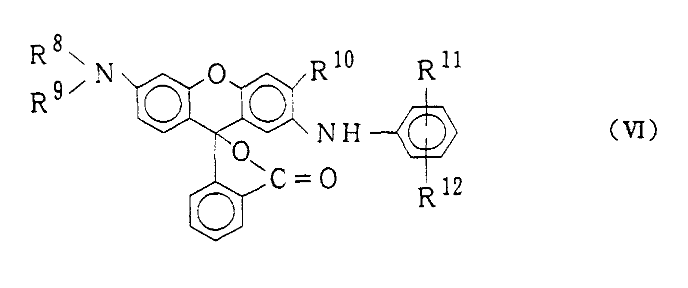 (wherein R⁸ and R⁹ independently represent an alkyl group, an alkoxyalkyl group, a cycloalkyl group, an aryl group or a tetrahydrofurfuryl group; R¹⁰ represents hydrogen, methyl or chlorine; R¹¹ and R¹² independently represent hydrogen, an alkyl group, chlorine or fluorine; R⁸ and R⁹ may, taken together with the adjacent nitrogen atom, form a heterocyclic group).

The following is a partial listing of fluoran compounds of general formula (VI) which can be employed.

2-Anilino-3-methyl-6-dimethylaminofluoran, 2-anilino-3-methyl-6-diethylaminofluoran, 2-anilino-3-methyl-6-di-n-propylaminofluoran, 2-anilino-3-methyl-6-di-n-butylaminofluoran, 2-anilino-3-methyl-6-di-n-amylaminofluoran, 2-anilino-3-methyl-6-(N-methyl-N-ethylamino)fluoran, 2-anilino-3-methyl-6-(N-methyl-N-n-propylamino)fluoran, 2-anilino-3-methyl-6-(N-methyl-N-n-butylamino)fluoran, 2-anilino-3-methyl-6-(N-methyl-N-isobutylamino)fluoran, 2-anilino-3-methyl-6-(N-methyl-N-n-amylamino)fluoran, 2-anilino-3-methyl-6-(N-methyl-N-cyclohexylamino)fluoran, 2-anilino-3-methyl-6-(N-ethyl-N-n-propylamino)fluoran, 2-anilino-3-methyl-6-(N-ethyl-N-n-butylamino)fluoran, 2-anilino-3-methyl-6-(N-ethyl-N-isobutylamino)fluoran, 2-anilino-3-methyl-6-(N-ethyl-N-n-amylamino)fluoran, 2-anilino-3-methyl-6-(N-ethyl-N-isoamylamino)fluoran, 2-anilino-3-methyl-6-(N-ethyl-N-n-octylamino)fluoran, 2-anilino-3-methyl-6-[N-ethyl-N-(3-ethoxypropyl)amino]fluoran, 2-anilino-3-methyl-6-(N-ethyl-N-p-tolylamino)fluoran, 2-anilino-3-methyl-6-(N-ethyl-N-tetrahydrofurfurylamino)fluoran, 2-anilino-3-chloro-6-diethylaminofluoran, 2-(2-chloroanilino)-6-diethylaminofluoran, 2-(2-chloroanilino)-6-di-n-butylaminofluoran, 2-(2-fluoroanilino)-6-diethylaminofluoran, 2-(3-trifluoromethylanilino)-6-diethylaminofluoran, 2-(3-methylanilino)-3-methyl-6-diethylaminofluoran, 2-(4-methylanilino)-3-methyl-6-diethylaminofluoran, 2-(4-t-amylanilino)-3-methyl-6-diethylaminofluoran, 2-(3-chloro-4-methylanilino)-3-methyl-6-diethylaminofluoran, 2-(2,4-dimethylanilino)-3-methyl-6-diethylaminofluoran, 2-(2,6-dimethylanilino)-3-methyl-6-diethylaminofluoran, 2-(2,6-diethylanilino)-3-methyl-6-diethylaminofluoran, 2-(2,4-dimethylanilino)-3-methyl-6-di-n-butylaminofluoran, 2-(2,6-dimethylanilino)-3-methyl-6-di-n-butylaminofluoran and 2-(2,6-diethylanilino)-3-methyl-6-di-n-butylaminofluoran.

In addition to the above combination of the compound of the invention with the fluoran compound (VI), any other leuco dye that is conventionally used in a recording material of this type can be incorporated in a suitable amount for adjusting the tone of the color to be developed.

When the compound of the invention and the fluoran compound of general formula (VI) are thus used in combination or when a different dye is further incorporated, the preferred proportion of the compound of the invention is not less than 20% by weight based on the total weight of the leuco dyes. When the proportion is less than 20% by weight, it may happen that the recorded image can hardly be read with an OCR.

The pressure-sensitive recording material can be manufactured by a variety of known methods as disclosed, inter alia, in Japanese Tokkyo Koho S-42-20144/1967. As a general procedure, the leuco dye is first dissolved in a microencapsulation solvent and the solution is microencapsulated by a known procedure, for example coacervation, interfacial polymerization, in situ polymerization or the like, using a high molecular compound as a wall material. The resulting dispersion of microcapsules is then coated on the reverse side of a support such as wood-free paper, synthetic paper or plastic film to provide an upper leaf. On the other hand, the color developer is coated on the face side of another support sheet to provide a lower leaf. The upper leaf is superposed on the lower leaf with the coated sides facing each other and the assembly is compressed imagewise, whereby the microcapsules in the compressed areas are destroyed and the leuco dye contained in the microcapsules is allowed to react with the color developer to form an image on the face side of the under leaf. Moreover, when a plurality of intermediate leaves each coated with the color developer on the face side of a support and the microcapsules on the reverse side are interposed between the upper leaf and the lower leaf, a plurality of transcriptions can be obtained.

The color developers that can be used in the pressure-sensitive recording material include acid clay, zinc salts of salicylic acid derivatives, zinc-modified p-octylphenol-formaldehyde resin, p-phenylphenol resin and so on. The preferred color developers are zinc salts of salicylic acid derivatives and zinc-modified p-octylphenol-formaldehyde resin.

The thermal recording material can be manufactured by a variety of known methods as disclosed in Japanese Tokkyo Koho S-45-14039, for instance. Generally speaking, the leuco dye, color developer and sensitizer are respectively dispersed in an aqueous solution of a water-soluble polymer such as poly(vinyl alcohol) by means of an attritor, sand mill or the like until the reagent will be finely divided to a particle diameter not more than a few microns. The sensitizer may be added to either the leuco dye or the color developer or to both and dispersed simultaneously. In certain cases an eutectic mixture with the leuco dye or the color developer may be prepared and dispersed. These dispersions are mixed and a pigment, binder, wax, metal soap, antioxidant, ultraviolet absorber, etc. are added as necessary to provide a heat-sensitive coating composition. This coating composition is coated on a support such as wood free paper, synthetic paper or plastic film and calendered for smoothing to provide a thermal recording material. For an improved quality of color, the heat-sensitive coating composition can be coated on a support having a precoat layer containing a plastic pigment or a heat insulating material such as silica. Moreover, where necessary, there may be formed an overcoat layer from an aqueous solution of a water-soluble polymer on the thermal recording layer for imparting resistance to water and chemicals.

The color developer which can be used in such a thermal recording material include a variety of phenolic compounds. Thus, bisphenol A, 2,2-bis(p-hydroxyphenyl)-4-methylpentane, 1,1-bis(p-hydroxyphenyl)cyclohexane, bisphenol S, 4-hydroxy-4'-isopropoxydiphenyl sulfone, 3,3'-diallyl-4,4'-dihydroxydiphenyl sulfone, 1,5-bis(p-hydroxyphenylmercapto)-3-oxapentane, benzyl p-hydroxybenzoate, tetrabromobisphenol A, tetrabromobisphenol S, etc. can be mentioned by way of example. Particularly preferred is bisphenol A.

The sensitizer includes but is not limited to p-benzylbiphenyl, m-terphenyl, 2-benzyloxynapthalene, 1,4-dibenzyloxynaphthalene, dibenzyl oxalate, di-(p-methylbenzyl) oxalate, di-(p-chlorobenzyl) oxalate, 1,2-diphenoxyethane, 1,2-di-m-toluoxyethane, 1,2-di-p-toluoxyethane, 1,4-diphenoxybutane, benzyl p-benzyloxybenzoate, phenyl 1-hydroxy-2-naphthoate, dibenzyl terephthalate, etc. Particularly preferred are p-benzylbiphenyl, m-terphenyl, 2-benzyloxynaphthalene, di-(p-methylbenzyl) oxalate and 1,2-di-m-toluoxyethane.

As the pigment, both organic and inorganic pigments can be employed. Preferred species are calcium carbonate, barium sulfate, titanium dioxide, aluminum hydroxide, amorphous silica, urea-formaldehyde resin powder, polyethylene resin powder and so on.

The binder can be a water-soluble polymer or a water-insoluble polymer. Preferred species of the water-soluble polymer include methylcellulose, carboxymethylcellulose, hydroxyethylcellulose, starch, styrene-maleic anhydride copolymer hydrolysate, ethylene-maleic anhydride copolymer hydrolysate, isobutylene-maleic anhydride copolymer hydrolysate, poly(vinyl alcohol), carboxyl-modified poly(vinyl alcohol), polyacrylamide, etc. and preferred species of the water-insoluble polymer include styrene-butadiene rubber latex, acrylonitrile-butadiene rubber latex, vinyl acetate emulsion and so on.

The wax includes paraffin wax, carboxyl-modified paraffin wax and polyethylene wax, to name but a few preferred examples.

The metal soap may be a higher fatty acid metal salt. Preferred species are zinc stearate, calcium stearate, aluminum stearate and the like.

The antioxidant may be a hindered phenol. The ultraviolet absorber may be a UV absorber in the benzophenone series or in the benzotriazole series.

The following examples are intended to describe the present invention in further detail and should by no means be construed as defining the scope of the invention. Examples 1, 3, 5 and 7 illustrate intermediates.

### Example 1 Production of α-crystal modification of 3-[2,2-bis(1-ethyl-2-methylindol-3-yl)ethenyl]-3-(4-diethylaminophenyl)phthalide

First, 34.2 g (0.1 mole) of 1,1-bis(1-ethyl-2-methylindol-3-yl)ethylene, 29.7 g (0.1 mole) of 2-(4-diethylaminobenzoyl)benzoic acid, 20.4 g (0.2 mole) of acetic anhydride and 50 mL of toluene were stirred at 60-65°C for 4 hours. After cooling, 250 mL of toluene and 250 g of 10% aqueous sodium hydroxide solution were added. The mixture was stirred at reflux for 1 hour, after which the toluene layer was taken, washed with warm water, filtered and concentrated. The residue was stirred with 200 mL of methanol at reflux for 1 hour. After cooling, the precipitate was recovered by filtration and dried to provide 49.6 g (yield 80%) of the α-crystal modification as a white powder. m.p. 127-130°C. In powder X-ray diffraction analysis, this α-crystal modification showed characteristic peaks at the diffraction angles (2 θ ± 0.2°) of 11.2°, 11.7°, 16.3°, 16.5°, 18.0° and 23.5°. The mass spectrum of this product is shown below.
MS (m/z): 621 (M⁺)

| Elemental analysis: | | | |
|---|---|---|---|
| | C | H | N |
| Calcd. (C₄₂H₄₃N₃O₂) | 81.11% | 6.98% | 6.76% |
| Found | 81.04% | 6.96% | 6.60% |

The 1,1-bis(1-ethyl-2-methylindol-3-yl)ethylene used in this example was synthesized in the following manner.

First, 318 g (2 moles) of 1-ethyl-2-methylindole, 78.5 g (1 mole) of acetyl chloride and 300 mL of acetic anhydride were stirred together at 50-55°C for 4 hours. Then, the reaction mixture was poured in 2L of water, made basic with sodium hydroxide and extracted with 2L of toluene. The toluene solution was washed with warm water, filtered and concentrated. The residue was stirred with 2L of methanol at reflux for 1 hour.
After cooling, the precipitate was collected by filtration and dried to provide 325 g (yield 95%) of a light brown powder. m.p. 179-181°C. This product was identified as the object ethylene compound based on the following analyses.
MS (m/z): 342 (M⁺)

| Elemental analysis: | | | |
|---|---|---|---|
| | C | H | N |
| Calcd. (C₂₄H₂₆N₂) | 84.15% | 7.67% | 8.18% |
| Found | 84.17% | 7.63% | 8.14% |

The 2-(4-diethylaminobenzoyl)benzoic acid was synthesized by the following procedure.

To a mixture of 300 g of chlorobenzene and 134 g of aluminum chloride under ice-cooling and stirring was added 149 g (1 mole) of diethylaniline dropwise followed by gradual addition of 74 g (0.5 mole) of phthalic anhydride. The mixture was stirred at 45-50°C for 4 hours. The reaction mixture was then taken into 1L of water and the chlorobenzene was distilled off. The precipitate was collected by filtration, washed with warm water, dried and recrystallized from methanol to provide 122.5 g (yield 82.5%) of a yellow-white powder. m.p. 183.5-184°C. This product was identified as the object benzoic acid derivative based on the following analyses.
MS (m/z): 297 (M⁺)

| Elemental analysis: | | | |
|---|---|---|---|
| | C | H | N |
| Calcd. (C₁₈H₁₉NO₃) | 72.69% | 6.45% | 4.71% |
| Found | 72.62% | 6.40% | 4.72% |

### Example 2-1 Production of β-crystal modification of 3-[2,2-bis(1-ethyl-2-methylindol-3-yl)ethenyl]-3-(4-diethylaminophenyl)phthalide

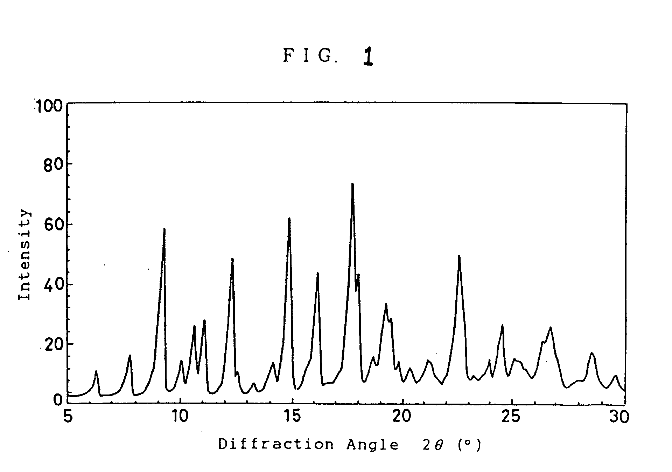

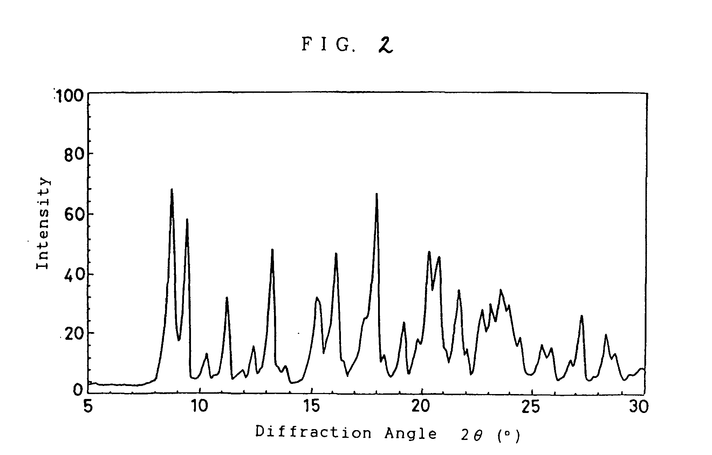

Twenty-five g of the α-crystal modification of 3-[2,2-bis(1-ethyl-2-methylindol-3-yl)ethenyl]-3-(4-diethylaminophenyl)phthalide produced in Example 1 and 100 mL of isopropyl alcohol were stirred at reflux for 1 hour. After cooling, the precipitate was recovered by filtration and dried to provide 23.4 g of the β-crystal modification as a white powder. m.p. 156-158°C. The Cu-Kα powder X-ray diffraction pattern of this β-crystal modification is shown in Fig. 2. In powder X-ray diffraction analysis, this β-crystal modification showed characteristic peaks at the diffraction angles (2 θ ± 0.2°) of 9.2°, 12.3°, 14.8°, 17.7°and 22.5°. The mass spectrum and elemental analysis of this product are presented below.
MS (m/z): 621 (M⁺)

| Elemental analysis: | | | |
|---|---|---|---|
| | C | H | N |
| Calcd. (C₄₂H₄₃N₃O₂) | 81.11% | 6.98% | 6.76% |
| Found | 81.18% | 6.95% | 6.70% |

### Example 2-2 Production of β-crystal modification of 3-[2,2-bis(1-ethyl-3-methylindol-3-yl)ethenyl]-3-(4-diethylaminophenyl)phthalide

Twenty-five (25) g of the α-crystal modification of 3-[2,2-bis(1-ethyl-2-methylindol-3-yl)ethenyl]-3-(4-diethylaminophenyl)phthalide produced in Example 1 and 100 mL of n-hexane were stirred at reflux for 1 hour. After cooling, the precipitate was recovered by filtration and dried to provide 24.0 g of the β-crystal modification as a white powder. m.p. 156-158°C. The Cu-Kα powder X-ray diffraction pattern of this β-crystal modification had characteristic peaks similar to those of the product obtained in Example 2-1. The identity of this product as the object compound could be further verified by mass spectrometry and elemental analysis.

### Example 2-3 Production of β-crystal modification of 3-[2,2-bis(1-ethyl-2-methylindol-3-yl)ethenyl]-3-(4-diethylaminophenyl)phthalide

First, 34.2 g (0.1 mole) of 1,1-bis(1-ethyl-2-methylindol-3-yl)ethylene, 29.7 g (0.1 mole) of 2-(4-diethylaminobenzoyl)benzoic acid, 20.4 g (0.2 mole) of acetic anhydride and 50 mL of toluene were stirred at 60-65°C for 4 hours. After cooling, 250 mL of toluene and 250 g of 10% aqueous sodium hydroxide solution were added. The mixture was stirred at reflux for 1 hour, after which the toluene layer was taken, washed with warm water, filtered and concentrated. The residue was stirred with 200 mL of isopropyl alcohol at reflux for 1 hour. After cooling, the precipitate was recovered by filtration and dried to provide 48.8 g of the β-crystal modification as a white powder. m.p. 156-158°C. The Cu-Kα powder X-ray diffraction pattern of this β-crystal modification had characteristic peaks similar to those of the product obtained in Example 2-1. The identity of this product as the object compound could be further verified by mass spectrometry and elemental analysis.

### Example 2-4 Production of β-crystal modification of 3-[2,2-bis(l-ethyl-2-methylindol-3-yl)ethenyl]-3-(4-diethylaminophenyl)phthalide

First, 31.8 g (0.2 mole) of 1-ethyl-2-methylindole, 7.85 g (0.1 mole) of acetyl chloride and 30 mL of acetic anhydride were stirred together at 50-55°C for 4 hours. Then, 17.6 g (0.1 mole) of calcium acetate monohydrate, 29.7 g (0.1 mol) of 2-(4-diethylaminobenzoyl)benzoic acid and 50 mL of toluene were added and the mixture was stirred at 60-65°C for 4 hours. After cooling, 250 mL of toluene and 250 g of 10% aqueous sodium hydroxide solution were added and the mixture was stirred at reflux for 1 hour. The toluene layer was then taken, washed with warm water, filtered and concentrated. The residue was stirred with 200 mL of isopropyl alcohol at reflux for 1 hour. After cooling, the precipitate was recovered by filtration and dried to provide 48.1 g (yield 77%) of the β-crystal modification as a white powder. m.p. 156-158°C. The Cu-Kα powder X-ray diffraction pattern of this β-crystal modification had characteristic peaks similar to those of the product obtained in Example 2-1. The identity of this product as the object compound could be further verified by mass spectrometry and elemental analysis.

### Example 3 Production of α-crystal modification of 3-[2,2-bis(1-ethyl-2-phenylindol-3-yl)ethenyl]-3-(4-dimethylaminophenyl)phthalide

First, 46.6 g (0.1 mole) of 1,1-bis(1-ethyl-2-phenylindol-3-yl)ethylene, 26.9 g (0.1 mole) of 2-(4-dimethylaminobenzoyl)benzoic acid, 20.4 g (0.2 mole) of acetic anhydride and 50 mL of toluene were stirred at 60-65°C for 4 hours. After cooling, 250 mL of toluene and 250 g of 10% aqueous sodium hydroxide solution were added. The mixture was stirred at reflux for 1 hour, after which the toluene layer was taken, washed with warm water, filtered and concentrated. The residue was stirred with 200 mL of methanol at reflux for 1 hour. After cooling, the precipitate was recovered by filtration and dried to provide 58.9 g (yield 82.1%) of a pale yellow-white powder. m.p. 133-135°C. In powder X-ray diffraction analysis, this α-crystal modification showed characteristic peaks at the diffraction angles (2 θ ± 0.2°) of 8.6° and 9.8°. The mass spectrum and elemental analysis of this product are shown below.
MS (m/z): 717 (M⁺)

| Elemental analysis: | | | |
|---|---|---|---|
| | C | H | N |
| Calcd. (C₅₀H₄₃N₃O₂) | 83.64% | 6.05% | 5.85% |
| Found | 83.70% | 6.17% | 5.76% |

The 1,1-bis(1-ethyl-2-phenylindol-3-yl)ethylene used in this example was synthesized in the following manner.

First, 442 g (2 moles) of 1-ethyl-2-phenylindole, 78.5 g (1 mole) of acetyl chloride and 300 mL of acetic anhydride were stirred together at 50-55°C for 20 hours. The reaction mixture was then poured in 2L of water and made basic with sodium hydroxide. The precipitate was recovered by filtration and, after washing with water, stirred in 2L of isopropyl alcohol at reflux for 1 hour. After cooling, the precipitate was collected by filtration and dried to provide 415 g (yield 89.1%) of a light brown powder. m.p. 165-167°C. This product was identified as the object ethylene compound based on the following analyses.
MS (m/z): 466 (M⁺)

| Elemental analysis: | | | |
|---|---|---|---|
| | C | H | N |
| Calcd. (C₃₄H₃₀N₂) | 87.50% | 6.49% | 6.00% |
| Found | 87.62% | 6.55% | 6.00% |

### Example 4-1 Production of β-crystal modification of 3-[2,2-bis(1-ethyl-2-phenylindol-3-yl)ethenyl]-3-(4-dimethylaminophenyl)phthalide

Twenty-five g of the α-crystal modification of 3-[2,2-bis(1-ethyl-2-phenylindol-3-yl)ethenyl]-3-(4-dimethylaminophenyl)phthalide produced in Example 3 and 100 mL of isopropyl alcohol were stirred at reflux for 1 hour. After cooling, the precipitate was recovered by filtration and dried to provide 23.9 g of a white powder. m.p. 195-196°C. The Cu-Kα powder X-ray diffraction pattern of this β-crystal modification is shown in Fig. 2. In powder X-ray diffraction analysis, this β-crystal modification showed characteristic peaks at the diffraction angles (2 θ ± 0.2°) of 8.7°, 9.4°, 13.2°, 17.9°and 20.3°. The mass spectrum and elemental analysis of this product are presented below.
MS (m/z): 717 (M⁺)

| Elemental analysis: | | | |
|---|---|---|---|
| | C | H | N |
| Calcd. (C₅₀H₄₃N₃O₂) | 83.64% | 6.05% | 5.85% |
| Found | 83.68% | 6.09% | 5.69% |

### Example 4-2 Production of β-crystal modification of 3-[2,2-bis(1-ethyl-2-phenylindol-3-yl)ethenyl]-3-(4-dimethylaminophenyl)phthalide

First, 44.2 g (0.2 mole) of 1-ethyl-2-phenylindole, 7.85 g (0.1 mole) of acetyl chloride and 30 mL of acetic anhydride were stirred together at 55-60°C for 20 hours. Then, 17.6 g (0.1 mole) of calcium acetate monohydrate, 26.9 g (0.1 mol) of 2-(4-dimethylaminobenzoyl)benzoic acid and 50 mL of toluene were added and the mixture was stirred at 60-65°C for 4 hours. After cooling, 250 mL of toluene and 250 g of 10% aqueous sodium hydroxide solution were added and the mixture was stirred at reflux for 1 hour. The toluene layer was then taken, washed with warm water, filtered and concentrated. The residue was stirred with 200 mL of isopropyl alcohol at reflux for 1 hour. After cooling, the precipitate was recovered by filtration and dried to provide 50.7 g (yield 70.7%) of a white powder. m.p. 196-200°C. The Cu-Kα powder X-ray diffraction pattern of this β-crystal modification had characteristic peaks similar to those of the product obtained in Example 4-1. The identity of this product as the object compound could be further verified by mass spectrometry and elemental analysis.

### Example 5 Production of α-crystal modification of 3-[2,2-bis(1-ethyl-2-phenylindol-3-yl)ethenyl]-3-(4-diethylaminophenyl)phthalide

First, 46.6 g (0.1 mole) of 1,1-bis(1-ethyl-2-phenylindol-3-yl)ethylene, 29.7 g (0.1 mole) of 2-(4-dimethylaminobenzoyl)benzoic acid, 20.4 g (0.2 mole) of acetic anhydride and 50 mL of toluene were stirred at 60-65°C for 4 hours. After cooling, 250 mL of toluene and 250 g of 10% aqueous sodium hydroxide solution were added. The mixture was stirred at reflux for 1 hour, after which the toluene layer was taken, washed with warm water, filtered and concentrated. The residue was stirred with 200 mL of methanol at reflux for 1 hour. After cooling, the precipitate was recovered by filtration and dried to provide 57.2 g (yield 76.8%) of a pale yellow-white powder. m.p. 163-165°C. In powder X-ray diffraction analysis, this α-crystal modification showed characteristic peaks at the diffraction angles (2 θ ± 0.2°) of 7.8°, 8.5° and 8.9°. The mass spectrum and elemental analysis of this product is shown below.
MS (m/z): 745 (M⁺)

| Elemental analysis: | | | |
|---|---|---|---|
| | C | H | N |
| Calcd. (C₅₂H₄₇N₃O₂) | 83.71% | 6.36% | 5.63% |
| Found | 83.65% | 6.27% | 5.56% |

### Example 6-1 Production of β-crystal modification of 3-[2,2-bis(1-ethyl-2-phenylindol-3-yl)ethenyl]-3-(4-diethylaminophenyl)phthalide

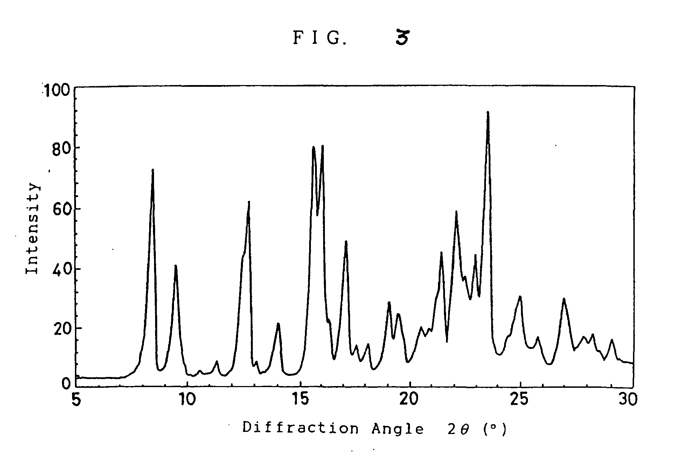

Twenty-five g of the α-crystal modification of 3-[2,2-bis(1-ethyl-2-phenylindol-3-yl)ethenyl]-3-(4-diethylaminophenyl)phthalide produced in Example 5 and 100 mL of isopropyl alcohol were stirred at reflux for 1 hour. After cooling, the precipitate was recovered by filtration and dried to provide 22.7 g of a pale yellow-white powder. m.p. 178-180°C. The Cu-Kα powder X-ray diffraction pattern of this β-crystal modification is shown in Fig. 3. In powder X-ray diffraction analysis, this β-crystal modification showed characteristic peaks at the diffraction angles (2 θ ± 0.2°) of 8.5°, 12.8°, 15.6°, 16.0°and 23.5°. The mass spectrum and elemental analysis of this product are presented below.
MS (m/z): 745 (M⁺)

| Elemental analysis: | | | |
|---|---|---|---|
| | C | H | N |
| Calcd. (C₅₂H₄₇N₃O₂) | 83.71% | 6.36% | 5.63% |
| Found | 83.60% | 6.38% | 5.51% |

### Example 6-2 Production of β-crystal modification of 3-[2,2-bis(1-ethyl-2-phenylindol-3-yl)ethenyl]-3-(4-diethylaminophenyl)phthalide

First, 44.2 g (0.2 mole) of 1-ethyl-2-phenylindole, 7.85 g (0.1 mole) of acetyl chloride and 30 mL of acetic anhydride were stirred together at 55-60°C for 20 hours. Then, 17.6 g (0.1 mole) of calcium acetate monohydrate, 29.7 g (0.1 mol) of 2-(4-diethylaminobenzoyl)benzoic acid and 50 mL of toluene were added and the mixture was stirred at 60-65°C for 4 hours. After cooling, 250 mL of toluene and 250 g of 10% aqueous sodium hydroxide solution were added and the mixture was stirred at reflux for 1 hour. The toluene layer was then taken, washed with warm water, filtered and concentrated. The residue was stirred with 200 mL of isopropyl alcohol at reflux for 1 hour. After cooling, the precipitate was recovered by filtration and dried to provide 50.2 g (yield 67.4%) of a pale yellow-white powder. m.p. 180-183°C. The Cu-Kα powder X-ray diffraction pattern of this β-crystal modification had characteristic peaks similar to those of the product obtained in Example 6-1. The identity of this product as the object compound could be further verified by mass spectrometry and elemental analysis.

### Example 7 Production of α-crystal modification of 3-[2,2-bis(1-ethyl-2-phenylindol-3-yl)ethenyl]-3-(4-di-n-propylaminophenyl)phthalide

First, 46.6 g (0.1 mole) of 1,1-bis(1-ethyl-2-phenylindol-3-yl)ethylene, 32.5 g (0.1 mole) of 2-(4-di-n-propylaminobenzoyl)benzoic acid, 20.4 g (0.2 mole) of acetic anhydride and 50 mL of toluene were stirred at 60-65°C for 4 hours. After cooling, 250 mL of toluene and 250 g of 10% aqueous sodium hydroxide solution were added. The mixture was stirred at reflux for 1 hour, after which the toluene layer was taken, washed with warm water, filtered and concentrated. The residue was stirred with 200 mL of methanol at reflux for 1 hour. After cooling, the precipitate was recovered by filtration and dried to provide 59.8 g (yield 77.4%) of a pale yellow-white powder. m.p. 180-185°C. In powder X-ray diffraction analysis, this α-crystal modification showed characteristic peaks at the diffraction angles (2 θ ± 0.2°) of 8.9° and 9.6°. The mass spectrum and elemental analysis of this product are shown below.
MS (m/z): 773 (M⁺)

| Elemental analysis: | | | |
|---|---|---|---|
| | C | H | N |
| Calcd. (C₅₄H₅₁N₃O₂) | 83.78% | 6.65% | 5.43% |
| Found | 83.95% | 6.71% | 5.34% |

### Example 8-1 Production of β-crystal modification of 3-[2,2-bis(1-ethyl-2-phenylindol-3-yl)ethenyl]-3-(4-di-n-propylaminophenyl]phthalide

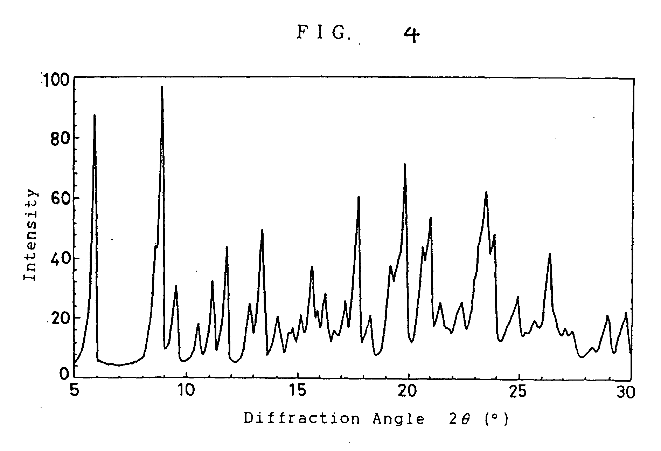

Twenty-five g of the α-crystal modification of 3-[2,2-bis(1-ethyl-2-phenylindol-3-yl)ethenyl]-3-(4-di-n-propylaminophenyl)phthalide produced in Example 7 and 100 mL of isopropyl alcohol were stirred at reflux for 1 hour. After cooling, the precipitate was recovered by filtration and dried to provide 24.1 g of a pale yellow-white powder. m.p. 186-188°C. The Cu-Kα powder X-ray diffraction pattern of this β-crystal modification is shown in Fig. 4. In powder X-ray diffraction analysis, this β-crystal modification showed characteristic peaks at the diffraction angles (2 θ ± 0.2°) of 5.9°, 8.8°, 17.7°, 19.7°and 23.4°. The mass spectrum and elemental analysis of this product are presented below.
MS (m/z): 773 (M⁺)

| Elemental analysis: | | | |
|---|---|---|---|
| | C | H | N |
| Calcd. (C₅₄H₅₁N₃O₂) | 83.78% | 6.65% | 5.43% |
| Found | 83.38% | 6.67% | 5.29% |

### Example 8-2 Production of β-crystal modification of 3-[2,2-bis(1-ethyl-2-phenylindol-3-yl)ethenyl]-3-(4-di-n-propylaminophenyl)phthalide

First, 44.2 g (0.2 mole) of 1-ethyl-2-phenyl-indole, 7.85 g (0.1 mole) of acetyl chloride and 30 mL of acetic anhydride were stirred together at 55-60°C for 20 hours. Then, 17.6 g (0.1 mole) of calcium acetate monohydrate, 32.5 g (0.1 mol) of 2-(4-di-n-propylaminobenzoyl)benzoic acid and 50 mL of toluene were added and the mixture was stirred at 60-65°C for 4 hours. After cooling, 250 mL of toluene and 250 g of 10% aqueous sodium hydroxide solution were added and the mixture was stirred at reflux for 1 hour. The toluene layer was then taken, washed with warm water, filtered and concentrated. The residue was stirred with 200 mL of isopropyl alcohol at reflux for 1 hour. After cooling, the precipitate was recovered by filtration and dried to provide 50.7 g (yield 65.6%) of a pale yellow-white powder. m.p. 188-192°C. The Cu-Kα powder X-ray diffraction pattern of this α-crystal modification had characteristic peaks similar to those of the product obtained in Example 8-1. The identity of this product as the object compound could be further verified by mass spectrometry and elemental analysis.

### Examples 9-12 Production of a pressure-sensitive recording material

Three grams of each compound of the invention was dissolved in 47 g of KMC-113 (a solvent available from Kureha Chemical Industry Co., Ltd.) under heating. To 100 g of water was added 5 g of an emulsifier (anionic copolymer) and the mixture was adjusted to pH 4 with aqueous sodium hydroxide solution. To this was added the above-prepared solution as well as 10 g of a melamine-formaldehyde prepolymer and the mixture was emulsified until the size of oil droplets was reduced to 4 µm. The emulsion was heated to 60°C under agitation and further stirred at that temperature for 1 hour. After cooling to room temperature, the emulsion was adjusted to pH 7.5 with 25% aqueous ammonia to provide a dispersion of microencapsulated leuco dye.

Ten grams of the thus-prepared dispersion of microcapsules was thoroughly mixed with 2 g of wheat starch and 1 g of latex and the resulting composition was coated on a sheet of wood free paper weighing 50 g/m² in a thickness equivalent to 5 g (solids)/m² to provide a white upper leaf.

This upper leaf was superposed on a lower leaf having a zinc-modified p-octylphenol-formaldehyde resin layer, with the coated sides facing each other, and the assembly was subjected to typewriting, whereupon a clear green blue image was obtained on the lower leaf.

For the compounds of claims 1 to 4, the recorded image had an absorption maximum at 710, 739, 741 and 740 nm, respectively. It showed not less than 80% of absorbance, relative to the maximum absorption, of 620-760, 700-770, 700-780 and 700-780 nm, respectively.

The upper leaf obtained above was superposed on a lower leaf having a zinc salt of salicyclic acid derivative layer, with the coated sides facing each other, and the assembly was subjected to typewriting, whereupon a clear green blue image was obtained on the lower leaf. For the compounds of claims 1 to 4, this recorded image had an absorption maximum at 711, 738, 740 and 740 nm, respectively. It showed not less than 80% of absorbance, relative to the maximum absorption, of 640-760, 680-780, 680-785 and 680-780 nm, respectively.

The images obtained were invariably suited for reading with any OCR equipped with a light source having an emission wavelength within the range of 620-760 nm, and showed excellent storage stability.

### Examples 13-16 Production of a pressure-sensitive recording material

In 47 g of KMC-113 (a solvent available from Kureha Chemical) were dissolved, with heating, 1.5 g of each compound of the invention, as well as 1.5 g of 2-anilino-3-methyl-6-diethylaminofluoran, 2-(2,6-dimethylanilino)-3-methyl-6-diethylaminofluoran, 2-(3-methylanilino)-3-methyl-6-diethylaminofluoran and 2-(2,4-dimethylanilino)-3-methyl-6-diethylaminofluoran, respectively.
On the other hand, 5 g of an emulsifier (anionic copolymer) was added to 100 g of water and the mixture was adjusted to pH 4 with aqueous sodium hydroxide solution. To this was added the above-prepared solution as well as 10 g of a melamine-formaldehyde prepolymer and the mixture was emulsified until the size of oil droplets was reduced to 4 microns. The emulsion was then heated to 60°C under agitation and stirred at that temperature for 1 hour. After cooling to room temperature, the emulsion was adjusted to pH 7.5 with 25% aqueous ammonia to provide a dispersion of the microencapsulated leuco dye.

Ten grams of the thus-prepared dispersion of microcapsules was thoroughly mixed with 2 g of wheat starch and 1 g of latex and the resulting composition was coated on a sheet of wood free paper weighing 50 g/m² in a thickness equivalent to 5 g (solids)/m² to provide a white upper leaf.

This upper leaf was superposed on a lower leaf having a zinc-modified p-octylphenol-formaldehyde resin layer, with the coated sides facing each other, and the assembly was subjected to typewriting, whereupon a clear black image was obtained on the lower leaf. This recorded image was suited for reading with any OCR equipped with a light source having an emission wavelength within the range of 620-760 nm. Moreover, the images had excellent storage stability.

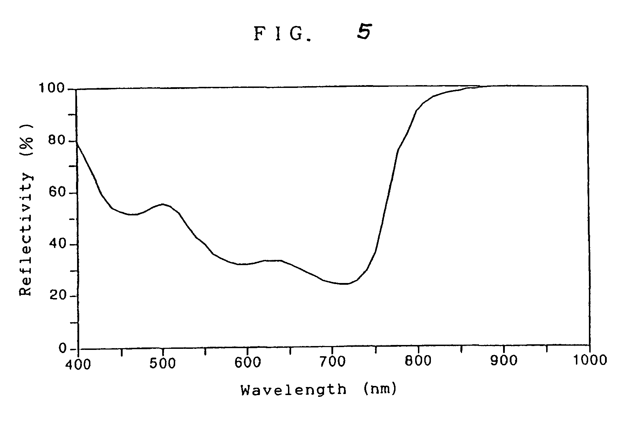

Fig. 5 shows the reflection spectrum of the colour image recorded on the lower leaf carrying a zinc-modified p-octylphenol-formaldehyde resin coat in a recording material of the invention (Example 13).

### Examples 17-20 Production of a thermal recording material

Five (5.0) grams of each compound of the invention, respectively, was milled with 45 g of 2.5% aqueous polyvinyl alcohol in a sand mill until the mean particle size was reduced to 1 µm, to provide a dispersion.

Separately, 10 g of bisphenol A and 10 g of p-benzylbiphenyl were milled with 80 g of 2.5% aqueous poly(vinyl alcohol) in a sand mill until the mean particle size was reduced to 1 micron to provide a dispersion.

These two dispersions were mixed well with 30 g of a 50% dispersion of calcium carbonate and 15 g of a 30% dispersion of paraffin wax to provide a heat-sensitive coating composition.

The thus-prepared coating composition was coated on a sheet of wood free paper weighing 50 g/m² in a thickness equivalent to 5 g (solids)/m², dried and calendered to a Bekk smoothness of 200 seconds on the recording layer to provide a white thermal recording material.

The background whiteness and background shelf-life of this thermal recording material were determined as follows.
Background whiteness: Using a reflection densitometer RD-914 (Macbeth Instrument Corporation), the degree of coloration of the background of each thermal recording material was determined as an optical density (OD) value.
Heat resistance of background: Each thermal recording material was exposed to 60°C/20% RH for 24 hours and the degree of background coloration was determined in the same manner as above.
Hot humidity resistance of background: Each thermal recording material was exposed to 50°C/90% RH for 24 hours and the degree of background coloration was determined in the same manner as above.

Light fastness of background: Each thermal recording material was irradiated with fluorescent light (2 x 10⁴ lux) for 72 hours and the degree of background coloration was determined in the same manner as above.

The results are shown in Table 1.

Furthermore, all the thermal recording materials were subjected to test recording with TF370 and Fax Test Chart 1. The resulting images were suited for reading with any OCR equipped with a light source having an emission wavelength within the range of 620 to 760 nm.

**[Table 1]**

| | | Background shelf-life | | |
|---|---|---|---|---|
| | Background whiteness (OD) | Heat resistance (OD) | Hot humidity resistance (OD) | Light fastness (OD) |
| Example 17 | 0.09 | 0.18 | 0.13 | 0.14 |
| Example 18 | 0.05 | 0.06 | 0.05 | 0.05 |
| Example 19 | 0.05 | 0.06 | 0.05 | 0.06 |
| Example 20 | 0.06 | 0.06 | 0.06 | 0.07 |
| Background whiteness: The smaller the value, the higher the degree of whiteness. Background shelf-life: The smaller the value after testing, the longer the shelf-life (higher storage stability). | | | | |

### Examples 21-24 Production of a thermal recording material

Using a sand mill, 2.5 g of each compound of the invention, respectively, 2.5 g of 2-(3-methylanilino)-3-methyl-6-diethylaminofluoran, 2-(anilino)-3-methyl-6-diethylaminofluoran, 2-(anilino)-3-methyl-6-(N-ethyl-N-tetrahydrofurfurylamino)fluoran and 2-(3-methylanilino)-3-methyl-6-di-n-butylaminofluoran, respectively, and 45 g of a 2.5% aqueous solution of polyvinyl alcohol were milled together until the mean particle size was reduced to 1 µm, to provide a dispersion.

Separately, 10 g of bisphenol A and 10 g of p-benzylphenyl were milled with 80 g of 2.5% aqueous PVA in a sand mill until the mean particle size was reduced to 1 µm, to provide a dispersion.

These two dispersions were mixed well with 30 g of a 50% dispersion of calcium carbonate and 15 g of a 30% dispersion of paraffin wax, to provide a heat-sensitive coating composition. This coating composition was coated on a sheet of wood-free paper weighing 50 g/m² in a thickness equivalent to 5 g (as solids)/m², dried and calendered to a Bekk smoothness of 200 seconds on the recording layer, to provide a white thermal recording material.

When recorded using Toshiba Facsimile TF370 and Fax Test chart No. 1, the thermal recording materials gave a black image. The image could be read with any OCR equipped with a light source having an emission wavelength within the range of 620-760 nm.

## Claims

1. A crystal form of 3-[2,2-bis(1-ethyl-2-methylindol-3-yl)ethenyl]-3-(4-diethylaminophenyl)phthalide, featuring a Cu-Kα powder X-ray diffraction pattern having characteristic peaks at the diffraction angles (2θ±0.2°) of 9.2°, 12.3°, 14.8°, 17.7° and 22.5°.

2. A crystal form of 3-[2,2-bis(1-ethyl-2-phenylindol-3-yl)ethenyl]-3-(4-dimethylaminophenyl)phthalide, featuring a Cu-Kα powder X-ray diffraction pattern having characteristic peaks at the diffraction angles (2θ ± 0.2°) of 8.7°, 9.4°, 13.2°, 17.9° and 20.3°.

3. A crystal form of 3-[2,2-bis(1-ethyl-2-phenylindol-3-yl)ethenyl]-3-(4-dlethylaminophenyl)phthalide, featuring a Cu-Kα powder X-ray diffraction pattern having characteristic peaks at the diffraction angles (2θ ± 0.2°) of 8.5°, 12.8°, 15.6°, 16.0° and 23.5°.

4. A crystal form of 3-[2,2-bis(1-ethyl-2-phenylindol-3-yl)ethenyl]-3-(4-di-n-propylaminophenyl)phthalide, featuring a Cu-Kα powder X-ray diffraction pattern having characteristic peaks at the diffraction angles (2θ ± 0.2°) of 5.9°, 8.8°, 17.7°, 19.7° and 23.4°.

5. A process for producing a crystal form according to any of claims 1 to 4, which comprises reacting a compound of formula (II) 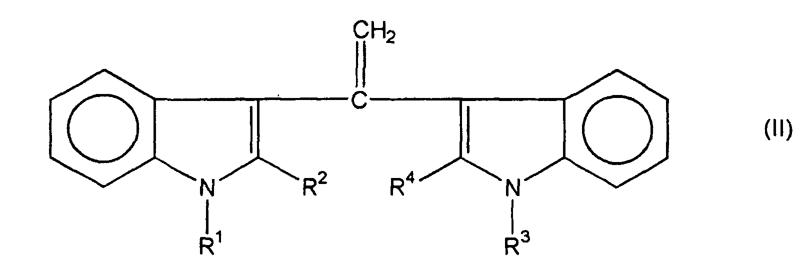 wherein R¹ and R³ are each ethyl, and R² and R⁴ are each methyl (for claim 1) or phenyl (for claims 2 to 4), with a compound of formula (III) 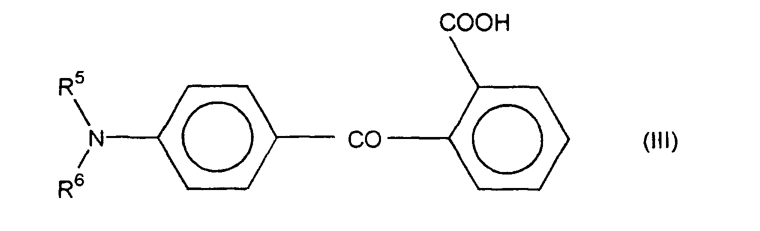 wherein R⁵ and R⁶ are each ethyl (for claims 1 and 3), methyl (for claim 2) or n-propyl (for claim 4); and heating the resulting reaction product with isopropyl alcohol or n-hexane.

6. A one-pot process for producing a crystal form according to any of claims 1 to 4, which comprises reacting an indole compound of the formula (IV) 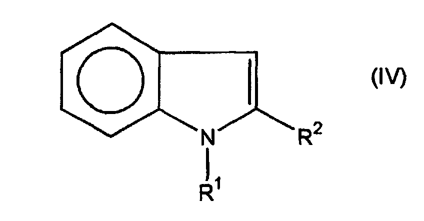 wherein R¹ and R² are as defined in claim 5, with acetyl chloride in acetic anhydride; adding a compound of formula (III) as defined in claim 5; and heating the reaction product with isopropyl alcohol or n-hexane.

7. A process according to claim 6, wherein the compound of formula (III) is added in the presence of sodium acetate, calcium acetate and/or calcium hydroxide.

8. A recording material utilizing the color-forming reaction between an electron-donating leuco dye and an electron-accepting color developer, wherein the electron-donating leuco dye comprises one or more crystal forms according to any of claims 1 to 4.

9. A recording material according to claim 8, wherein the electron-donating leuco dye comprises the one or more crystal forms in combination with one or more fluoran compounds of the formula (VI) 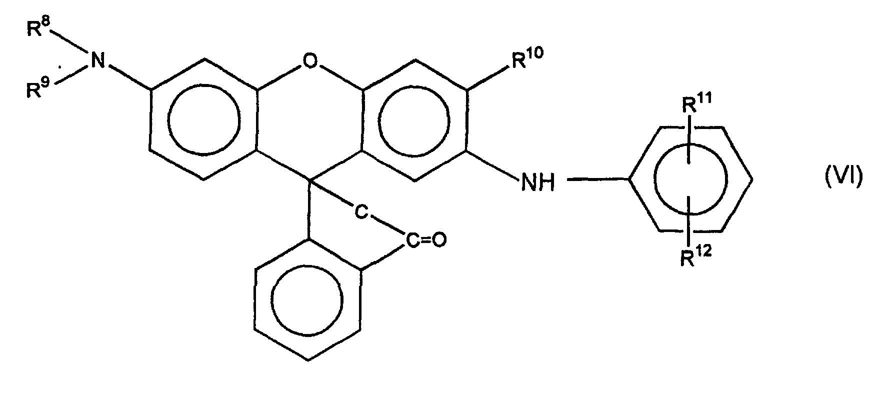 wherein R⁸ and R⁹ are independently alkyl, alkoxyalkyl, cycloalkyl, aryl or tetrahydrofurfuryl, or NR⁸R⁹ is a heterocyclic group; R¹⁰ is hydrogen, methyl or chloro; and R¹¹ and R¹² are independently hydrogen, alkyl, chloro or fluoro.

10. A recording material according to claim 8 or claim 9, which is a pressure-sensitive recording material.

11. A recording material according to claim 8 or claim 9, which is a thermal recording material.

## Patentansprüche

1. Kristallform von 3-[2,2-Bis(1-ethyl-2-methylindol-3-yl)ethenyl]-3-(4-diethylaminophenyl)phthalid, welche ein Cu-Kα Pulver-Röntgenbeugungsmuster mit charakteristischen Peaks bei den Beugungswinkeln (2θ ± 0,2°) 9,2°, 12,3°, 14,8°, 17,7° und 22,5° aufweist.

2. Kristallform von 3-[2,2-Bis(1-ethyl-2-phenylindol-3-yl)ethenyl]-3-(4-dimethylaminophenyl)phthalid, welche ein Cu-Kα Pulver-Röntgenbeugungsmuster mit charakteristischen Peaks bei den Beugungswinkeln (2θ ± 0,2°) 8,7°, 9,4°, 13,2°, 17,9° und 20,3° aufweist.

3. Kristallform von 3-[2,2-Bis(1-ethyl-2-phenylindol-3-yl)ethenyl]-3-(4-diethylaminophenyl)phthalid, welche ein Cu-Kα Pulver-Röntgenbeugungsmuster mit charakteristischen Peaks bei den Beugungswinkeln (2θ ± 0,2°) 8,5°, 12,8°, 15,6°, 16,0° und 23,5° aufweist.

4. Kristallform von 3-[2,2-Bis(1-ethyl-2-phenylindol-3-yl)ethenyl]-3-(4-di-npropylaminophenyl)phthalid, welche ein Cu-Kα Pulver-Röntgenbeugungsmuster mit charakteristischen Peaks bei den Beugungswinkeln (2θ ± 0,2°) 5,9°, 8,8°, 17,7°, 19,7° und 23,4° aufweist.

5. Verfahren zur Herstellung einer Kristallform gemäß einem der Ansprüche 1 bis 4, welches die Umsetzung einer Verbindung der Formel (II) 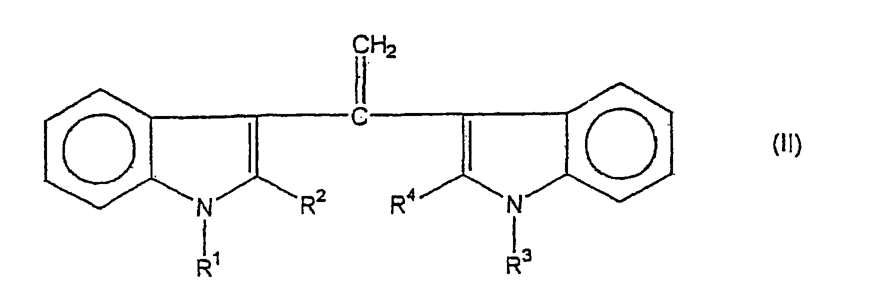 wobei R¹ und R³ jeweils eine Ethylgruppe, und R² und R⁴ jeweils eine Methyl- (für Anspruch 1) oder Phenylgruppe (für die Ansprüche 2 bis 4) darstellen, mit einer Verbindung der Formel (III) 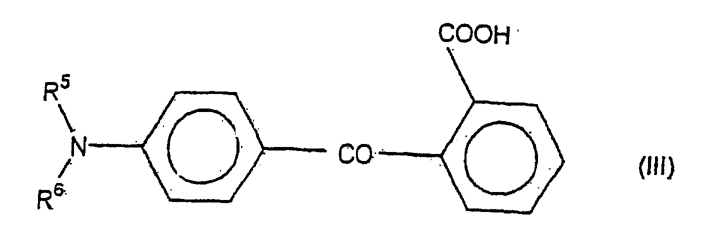 wobei R⁵ und R⁶ jeweils eine Ethyl- (für die Ansprüche 1 und 3), Methyl- (für Anspruch 2) oder n-Propylgruppe (für Anspruch 4) darstellen; und Erwärmen des erhaltenen Reaktionsprodukts mit Isopropylalkohol oder n-Hexan umfasst.

6. Eintopfverfahren zur Herstellung einer Kristallform gemäß einem der Ansprüche 1 bis 4, welches die Umsetzung einer Indolverbindung der Formel (IV) 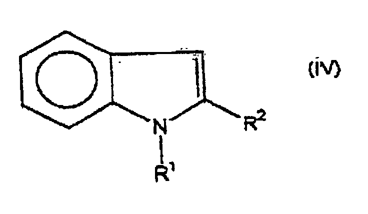 wobei R¹ und R² wie in Anspruch 5 definiert sind, mit Acetylchlorid in Essigsäureanhydrid; die Zugabe einer Verbindung der Formel (III), wie in Anspruch 5 definiert; und Erwärmen des Reaktionsprodukts mit Isopropylalkohol oder n-Hexan umfasst.

7. Verfahren gemäß Anspruch 6, wobei die Verbindung der Formel (III) in Gegenwart von Natriumacetat, Calciumacetat und/oder Calciumhydroxid zugegeben wird.

8. Aufzeichnungsmaterial, welches die Farbstoff-bildende Reaktion zwischen einem Elektronen-abgebenden Leukofarbstoff und einem Elektronen-aufnehmenden Farbentwickler verwendet, wobei der Elektronen-abgebende Leukofarbstoff eine oder mehrere Kristallformen gemäß einem der Ansprüche 1 bis 4 umfasst.

9. Aufzeichnungsmaterial gemäß Anspruch 8, wobei der Elektronen-abgebende Leukofarbstoff die eine oder mehreren Kristallformen in Kombination mit einer oder mehreren Fluoranverbindungen der Formel (VI) 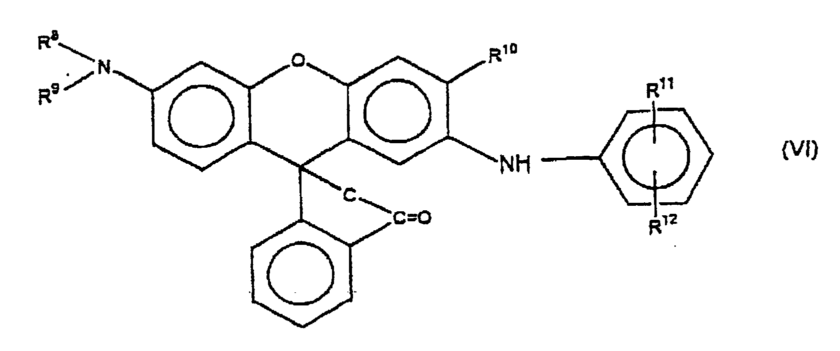 wobei R⁸ und R⁹ unabhängig voneinander ein Alkyl-, Alkoxyalkyl-, Cycloalkyl-, Aryl- oder Tetrahydrofurfurylrest sind, oder NR⁸R⁹ einen heterocyclischen Rest darstellen; R¹⁰ ein Wasserstoffatom, eine Methylgruppe oder ein Chloratom ist und R¹¹ und R¹² unabhängig voneinander ein Wasserstoffatom, ein Alkylrest, ein Chlor- oder Fluoratom sind, umfasst.

10. Aufzeichnungsmaterial gemäß Anspruch 8 oder Anspruch 9, welches ein auf Druck ansprechendes Aufzeichnungsmaterial ist.

11. Aufzeichnungsmaterial gemäß Anspruch 8 oder Anspruch 9, welches ein thermisches Aufzeichnungsmaterial ist.

## Revendications

1. Forme cristalline du 3-[2,2-bis(1-éthyl-2-méthylindol-3-yl)éthényl]-3-(4-diéthylaminophényl)phtalidure, donnant un diagramme de diffraction de poudre aux rayons X, raie Cu-Kα, ayant les pics caractéristiques aux angles de diffraction (2θ±-0,2°) de 9,2°, 12,3°, 14,8°, 17,7° et 22,5°.

2. Forme cristalline du 3-[2,2-bis(1-éthyl-2-phénylindol-3-yl)éthényl]-3-(4-diméthylaminophényl)phtalidure, donnant un diagramme de diffraction de poudre aux rayons X, raie Cu-Kα, ayant les pics caractéristiques aux angles de diffraction (2θ±0,2°) de 8,7°, 9,4°, 13,2°, 17,9°,et 20,3°.

3. Forme cristalline du 3-[2,2-bis(1-éthyl-2-phénylindol-3-yl)éthényl]-3-(4-diéthylaminophényl)phtalidure, donnant un diagramme de diffraction de poudre aux rayons X, raie Cu-Kα, ayant les pics caractéristiques aux angles de diffraction (2θ±0,2°) de 8,7°, 9,4°, 13,2°, 17,9°,et 20,3°.

4. Forme cristalline du 3-[2,2-bis(1-éthyl-2-phénylindol-3-yl)éthényl]-3-(4-di-n-propylaminophényl)phtalidure, donnant un diagramme de diffraction de poudre aux rayons X, raie Cu-Kα, ayant les pics caractéristiques aux angles de diffraction (2θ±0,2°) de 5,9°, 8,8°, 17,7°, 19,7°,et 23,4°.

5. Procédé pour produire une forme cristalline selon l'une quelconque des revendications 1 à 4, qui consiste à faire réagir un composé de formule (II) 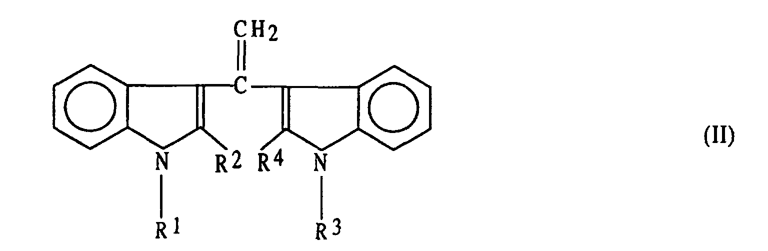 dans lequel R¹ et R³ sont chacun un éthyl et R² et R⁴ sont chacun un méthyl (pour la revendication 1) ou un phényl (pour les revendications 2 à 4) avec un composé de formule (III) 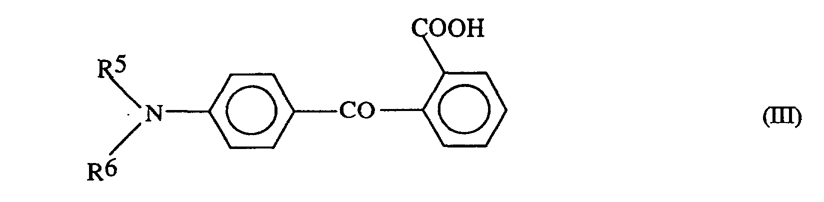 dans laquelle R⁵ et R⁶ sont chacun éthyle (pour les revendications 1 et 3), méthyle (pour les revendication 2) ou n-propyle (pour la revendication 4); et le chauffage du produit de réaction résultant avec l'alcool d' isopropyle ou du n-hexane.

6. Un procédé dans un seul réacteur pour la production d'une forme cristalline selon l'une quelconque des revendications 1 à 4, qui comprend la réaction d'un composé indole de la formule (IV) : 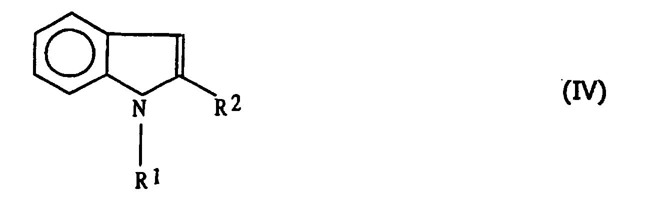 Dans laquelle R ¹ et R ² ont été définis à la revendication 5, avec du chlorure d'acétyle dans l'anhydride acétique ; l'ajout d'un composé de la formule (III) comme défini à la revendication 5 ; et le chauffage du produit de réaction avec de l'alcool d'isopropyle ou du n-hexane.

7. Procédé selon la revendication 6, dans lequel le composé de la formule (III) est ajouté en présence d'acétate de sodium, d'acétate de calcium et/ou d'hydroxyde de calcium.

8. Matériau d'enregistrement utilisant la réaction de formation de couleur entre un leuco-colorant donne des électrons et un développeur de couleur acceptant des électrons, dans lequel le leuco-colorant donnant des électrons comprend une ou plusieurs formes cristallines selon l'une quelconque des revendications 1 à 4.

9. Matériau d'enregistrement selon la revendication 8, dans lequel le leuco-colorant donneur d'électrons comprend une ou plusieurs formés cristallines en combinaison avec un ou plusieurs fluoranes de la formule (VI): 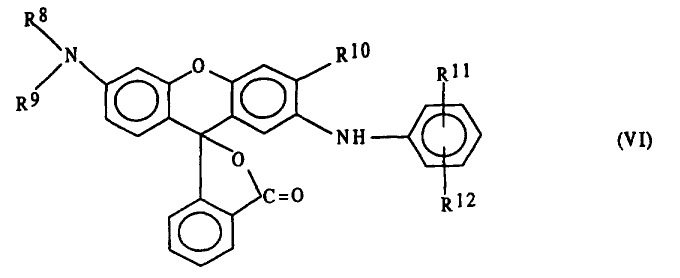 Dans laquelle R⁸ et R⁹ sont indépendamment alkyle, alkoxyalkyle, cycloalkyle, aryle ou tétrahydrofurfuryle, ou NR⁸ R⁹ est un groupe hétérocyclique ; R¹⁰ est l'hydrogène, méthyle ou chloro ; R¹¹ et R¹² sont indépendamment l'hydrogène, alkyle, chloro ou fluoro.

10. Un matériau d'enregistrement selon la revendication 8 ou 9, qui est un matériau d'enregistrement sensible à la pression.

11. Un matériau d'enregistrement selon la revendication 8 ou 9, qui est un matériau d'enregistrement thermique.
